(19) Europäisches Patentamt
European Patent Office
Office européen des brevets

(11) Publication number: **0 345 999 A1**

(12) **EUROPEAN PATENT APPLICATION**

(21) Application number: **89305514.5**

(51) Int. Cl.[4]: **C07D 205/08**

(22) Date of filing: **01.06.89**

(30) Priority: **06.06.88 US 202288**

(43) Date of publication of application: **13.12.89 Bulletin 89/50**

(84) Designated Contracting States: **AT BE CH DE ES FR GB GR IT LI NL SE**

(71) Applicant: **ELI LILLY AND COMPANY**
**Lilly Corporate Center**
**Indianapolis Indiana 46285(US)**

(72) Inventor: **Doecke, Christopher William**
**550 San Ricardo Drive**
**Greenwood Indiana 46242(US)**

(74) Representative: **Tapping, Kenneth George et al**
**Erl Wood Manor**
**Windlesham Surrey, GU20 6PH(GB)**

(54) **Process for intermediates to 1-carba(1-dethia)-3-cephem-4-carboxylic acids.**

(57) The present invention provides a process for preparing α-diazo-β-keto ester intermediates to 1-carba(1-dethia)-3-cephem-4-carboxylic acids.

EP 0 345 999 A1

# PROCESS FOR INTERMEDIATES TO 1-CARBA(1-DETHIA)-3-CEPHEM-4-CARBOXYLIC ACIDS

This invention relates to the synthesis of intermediates to 1-carba(1-dethia)-3-cephem-4-carboxylic acids and derivatives thereof. The process of the invention is preferably used to prepare intermediates to a class of potent orally active 1-carba(1-dethia)-3-cephem-4-carboxylic acid antibiotics described by J. Hashimoto et al. in U.S. Patent Nos. 4,335,211 and 4,708,956.

The 1-carba(1-dethia)-3-cephem-4-carboxylic acids, hereinafter 1-carbacephalosporins, possess the 4,6 bicyclic ring system represented by the following structural formula

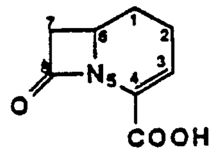

wherein the arbitrary numbering system employed according to the cephem nomenclature system is used as indicated.

The 1-carbacephalosporins thus far have not been obtained from natural sources, for example, as microbial metabolites. Accordingly, methods for the total synthesis of these promising compounds are highly desirable, particularly methods which are adaptable to large scale manufacture.

U.S. Patent No. 4,665,171 discloses a process for preparing 1-carbacephalosporins. More particularly, the patent discloses a process for preparing the compounds prepared by the present process employing p-toluenesulfonyl azide, an extremely dangerous compound, as the diazo transfer reagent. The patent teaches the isolation of the synthesized product by chromatography over silica. The process disclosed in the patent is not desirable for the synthesis of the compounds on a large scale.

The present invention provides a process for preparing an intermediate to 1-carbacephalosporins which is particularly well suited for a large scale industrial setting. More specifically, the present invention relates to a process for preparing a compound of the Formula I

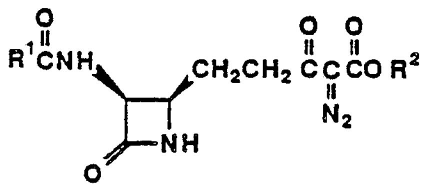

I

wherein $R^1$ is $C_1$-$C_6$ alkyl; a phenyl group

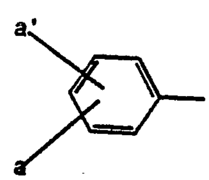

wherein a and a′ independently are hydrogen, $C_1$-$C_4$ alkyl, $C_1$-$C_4$ alkoxy or halogen; a group represented by the formula

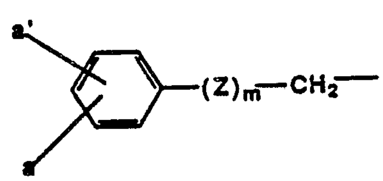

wherein Z is O or S, m is 0 or 1, and a and a′ have the same meanings as defined above; or $R^1$ is $R^{1'}O$ wherein $R^{1'}$ represents $C_1$-$C_4$ alkyl, $C_5$-$C_7$ cycloalkyl, benzyl, nitrobenzyl, methoxybenzyl, or halobenzyl; and $R^2$ is $C_1$-$C_4$ alkyl, allyl, 2,2,2-trichloroethyl, 2,2,2-tribromoethyl, β-tri($C_1$-$C_4$ alkyl)silylether, benzyl, $C_1$-$C_4$ alkylbenzyl, $C_1$-$C_4$ alkoxybenzyl, nitrobenzyl, or chlorobenzyl, comprising reacting a β-keto ester of the Formula II

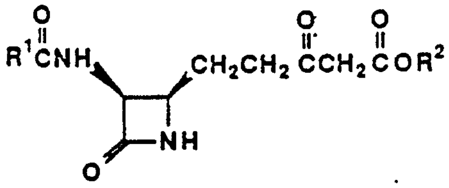

II

with a diazo transfer reagent of the Formula III

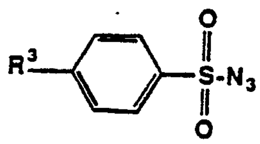

III

wherein $R^3$ is $C_8$-$C_{15}$ alkyl in a halogenated hydrocarbon solvent in the presence of a suitable base, and optionally isolating the compound of Formula I by the addition to the reaction mixture of a solvent in which the compound of Formula I is preferentially insoluble and the diazo transfer reagent is preferentially soluble.

All temperatures stated herein are in degrees Celsius. All quantities specified are in weight units, except for liquids, which are in volume units.

The term "$C_1$-$C_6$ alkyl" represents a straight or branched alkyl chain having from 1 to 6 carbon atoms. Typical $C_1$-$C_6$ alkyl groups include methyl, ethyl, n-propyl, isopropyl, n-butyl, sec.-butyl, t-butyl, n-pentyl, isopentyl, n-hexyl, isohexyl and the like.

$C_8$-$C_{15}$ Alkyl represents a straight or branched alkyl chain having from 8 to 15 carbon atoms. Typical $C_8$-$C_{15}$ alkyl groups include n-octyl, n-nonyl, n-decyl, n-dodecyl and the like. Of these, n-dodecyl is preferred.

Halogen represents fluoro, chloro, bromo or iodo.

$C_1$-$C_4$ Alkoxy represents a straight or branched alkoxy chain having from 1 to 4 carbon atoms. Typical $C_1$-$C_4$ alkoxy groups include methoxy, ethoxy, n-propoxy, isopropoxy, n-butoxy, isobutoxy and the like.

$C_5$-$C_7$ Cycloalkyl represents cyclopentyl, cyclohexyl or cycloheptyl.

While the entire scope of process variables taught herein are believed operable, the present process does have preferred aspects. In the above formulas, $R^2$ is preferable nitrobenzyl, and especially 4-nitrobenzyl. $R^1$ is preferably a group represented by the formula

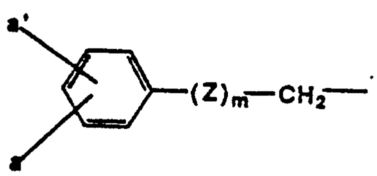

wherein a and a′ are hydrogen, m is 1 and Z is O. Other preferred aspects of the present invention will be noted hereinafter.

The process of the present invention is carried out as follows. Approximately one equivalent of a β-keto ester defined by Formula II above is slurried in a halogenated hydrocarbon solvent. Suitable halogenated hydrocarbon solvents include methylene chloride, 1,2-dichloroethane, chloroform, carbon tetrachloride, 1,1,1-trichloroethane, 1,1,2,2-tetrachloroethane, pentachloroethane and the like. Of these, methylene chloride is preferred. As in any chemical process the concentration of reactants in the reaction mixture is not critical, but it is preferred to employ the least amount of solvent necessary to keep the reactants in solution. The range of practical concentration depends in part on the power of the mixing equipment used, and in part on

the solubility of the reactants in the chosen solvent. Generally, it is convenient to conduct the reaction at a concentration in the range of about 500 ml to 1L of halogenated hydrocarbon solvent per gram mole of β-keto ester, with about 500 ml per gram mole being preferred. This concentration range is both economical and convenient in large scale production. Higher concentrations of reactants are feasible with the understanding that it will not be possible to carry out the desired smooth conversion of reactants from step to step if the starting materials are allowed to partially precipitate out of solution during the reaction period.

At least one equivalent of a suitable base is next added to the reaction mixture. Suitable bases include the tertiary amines such as triethlyamine, diisopropylethylamine, and the like, and other bases such as diazabicycloundecane (DBU).

After the reactants are thoroughly mixed, a diazo transfer reagent is combined with the other reactants. Typically, at least one mole equivalent of transfer reagent is employed for each mole equivalent of β-keto ester of Formula II employed. However, a slight excess of transfer reagent is preferred to ensure complete reaction of the β-keto ester starting material.

The process of the present invention is substantially complete after about 12 to 48 hours or more when conducted at a temperature in the range of about -25° C to about 100° C. The reaction is preferable conducted for approximately 24 hours at a temperature in the range of about 20° C to about 25° C.

As yet another embodiment of the present invention, the product of the present invention may be isolated by adding a solvent to the reaction mixture in which the compound of Formula I is preferentially insoluble and the diazo transfer reagent is preferentially soluble. Suitable solvents for use in this step of the process include the saturated aliphatic hydrocarbons such as pentane, cyclohexane, hexane, octane and heptane, which is preferred. The reaction mixture containing the chosen solvent is typically slurried until thoroughly mixed, and the precipitated solid collected by standard techniques such as vacuum filtration.

The compounds prepared by the present process may by further purified by dissolving the compound in a minimal amount of N,N-dimethylformamide (DMF). To this solution is added approximately an equivolume amount of water, and the resulting suspension is stirred at room temperature for a short period of time, typically less than two hours. The precipitated solid may then be easily isolated, such as by vacuum filtration, and the resulting solid washed and dried according to standard procedures to provide the desired compound in highly pure form.

The process of the present invention is particularly advantageous because it is capable of preparing the valuable 1-carbacephalosporin intermediates which are its products in high purity without the need for additional purification. Further, the process may be carried out under simplified conditions in an inexpensive manner, and as such the process is particularly well suited for the large scale industrial synthesis of the desired intermediate to the 1-carbacephalosporin product. Finally, since the product of the present process may be isolated without the use of expensive and laborious column chromatography procedures, the reaction is economically advantageous. This is especially important when using the present process in a large scale operation.

As noted above, the compounds prepared by the process of the present invention are useful as intermediates in the synthesis of 1-carbacephalosporins. For example, the compound of Formula I undergoes rhodium catalyzed cyclization to the corresponding 3-hydroxy-1-carbacephalosporin as taught by Evans et al. in U.S. Patent No. 4,665,171, herein incorporated by reference.

The starting materials employed in the present process are prepared by prior art procedures, or by processes analogous to such prior art procedures. For example, the β-keto esters of Formula II may be prepared by the procedures set forth by Evans et al. in U.S. Patent No. 4,665,171, herein incorporated by reference. The diazo transfer reagents employed in the present process may also be prepared by standard procedures. The preferred diazo transfer reagent, 4-dodecylbenzenesulfonyl azide, is a known compound readily prepared by published procedures. See, e.g., Hazen et al. in Synthetic Communications, 11(12), 947-956 (1981).

The following Example further illustrates specific aspects of the present invention. The Example is not intended to be limiting to the scope of the process of the invention in any respect and should not be so construed.

## Example 1

cis-α-Diazo-β,4-dioxo-3-[(phenoxyacetyl)amino]-2-azetidinepentanoic acid, (4-nitrophenyl)methyl ester

Five grams (10.7 mmol) of cis-β,4-dioxo-3-[(phenoxyacetyl)amino]-2-azetidinepentanoic acid, (4-nitrophenyl)methyl ester were slurried in 70 ml of methylene chloride. To the mixture was added 1.08 g (1.48 ml, 10.7 mmol) of triethylamine via syringe and the resulting mixture was stirred at room temperature for 10 minutes. Next, 4.12 g (11.7 mmol) of 4-dodecylbenzenesulfonyl azide was added to the mixture with 5 ml of methylene chloride. The mixture was stirred for 21 hours at room temperature and 100 ml of heptane was added dropwise over a 30 minute period. The mixture was stirred for several hours and the precipitated solid was collected by vacuum filtration. The solid was washed with heptane and dried in a vacuum oven at 50 °C to provide 4.48 g of cis-α-diazo-β,4-dioxo-3-[(phenoxyacetyl)amino]-2-azetidinepentanoic acid, (4-nitrophenyl)methyl ester. Yield 85%.

A portion of the compound thus prepared was further purified as follows. Forty milliliters of water were added dropwise to a solution of 4.08 g of cis-α-diazo-β,4-dioxo-3-[(phenoxyacetyl)amino]-2-azetidinepentanoic acid, (4-nitrophenyl)methyl ester in 40 ml of DMF over a period of fifty minutes. The resulting suspension was stirred at room temperature for an additional 45 minutes. The precipitated solid was collected by vacuum filtration and the flask was washed with 25 ml of DMF:water (1:1, v:v). The collected solid was washed with 25 ml of water, 25 ml of isopropyl alcohol, twice with 25 ml of hexanes and dried in an oven to provide 3.21 g of cis-α-diazo-β,4-dioxo-3-[(phenoxyacetyl)amino]-2-azetidinepentanoic acid, (4-nitrophenyl)methyl ester which was 100% pure by high pressure liquid chromatographic analysis.

## Claims

1. A process for preparing a compound of the Formula I

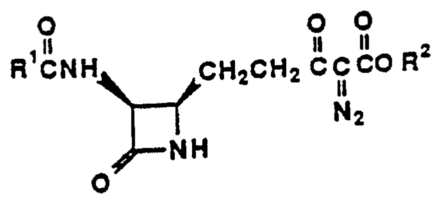

. I

wherein $R^1$ is $C_1$-$C_6$ alkyl; a phenyl group

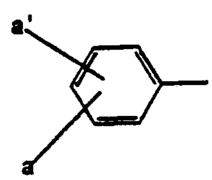

wherein a and a′ independently are hydrogen, $C_1$-$C_4$ alkyl, $C_1$-$C_4$ alkoxy or halogen; a group represented by the formula

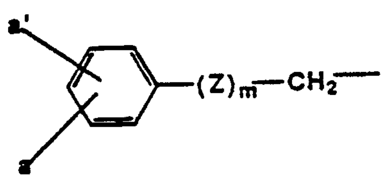

,

wherein Z is O or S, m is 0 or 1, and a and a′ have the same meanings as defined above; or $R^1$ is $R^{1'}O$ wherein $R^{1'}$ represents $C_1$-$C_4$ alkyl, $C_5$-$C_7$ cycloalkyl, benzyl, nitrobenzyl, methoxybenzyl, or halobenzyl; and $R^2$ is $C_1$-$C_4$ alkyl, allyl, 2,2,2-trichloroethyl, 2,2,2-tribromoethyl, β-tri($C_1$-$C_4$ alkyl)silylether, benzyl, $C_1$-$C_4$ alkylbenzyl, $C_1$-$C_4$ alkoxybenzyl, nitrobenzyl, or chlorobenzyl, comprising reacting a β-keto ester of the Formula II

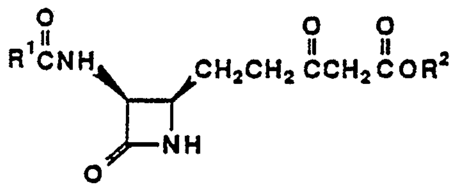

II

with a diazo transfer reagent of the Formula III

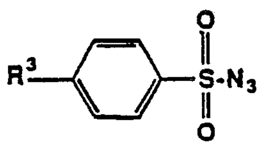

III

wherein $R^3$ is $C_8$-$C_{15}$ alkyl, in a halogenated hydrocarbon solvent in the presence of a suitable base, and optionally isolating the compound of Formula I by the addition of a solvent in which the compound of Formula I is preferentially insoluble and the diazo transfer reagent is preferentially soluble.

2. A process of Claim 1 wherein $R^1$ is phenoxy methylene.

3. A process of Claim 2 wherein $R^2$ is (4-nitrophenyl)methyl.

4. A process of Claim 1 wherein the diazo transfer agent is 4-dodecylbenzenesulfonyl azide.

5. A process of Claim 1 wherein the halogenated hydrocarbon solvent is methylene chloride.

6. A process of Claim 1 wherein the suitable base is triethylamine.

7. A process of Claim 1 wherein the suitable base is diazabicycloundecane.

8. A process of Claim 1 wherein the solvent in which the compound of Formula I is preferentially insoluble and the diazo transfer reagent is preferentially soluble is heptane.

European Patent Office

# EUROPEAN SEARCH REPORT

Application number
EP 89305514.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,Y | US - A - 4 665 171 (EVANS et al.) * Columns 9-11; examples 6A,B * -- | 1-6,8 | C 07 D 205/08 |
| D,Y | SYNTHETIC COMMUNICATIONS, 11(12), 1981 RHAZEN et al. "A safer diazotransfer reagent" pages 947-956 * Table II product (i), examples * -- | 1-6,8 | |
| A | JOURNAL OF THE AMERICAN CHEMICAL SOCIETY, vol. 102, no. 19, 1980 SALZMANN et al. "A stereo--controlled synthesis of (+)--thienamycin" pages 6161-6163 * Page 6162, scheme II; page 6163, left column: compounds 17,18 * ---- | 1-8 | |

| TECHNICAL FIELDS SEARCHED (Int. Cl.4) |
|---|
| C 07 D 205/00 |

The present search report has been drawn up for all claims

| Place of search | Date of completion of the search | Examiner |
|---|---|---|
| VIENNA | 30-08-1989 | JANISCH |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document